# EUROPEAN PATENT APPLICATION

(11) **EP 2 526 993 A2**
(43) Date of publication of application: **28.11.2012**
(21) Application number: 11734542.1
(22) Date of filing: 07.01.2011
(51) Int. Cl.: A61M 25/01, A61B 18/12, A61M 31/00

(54) **TREATMENT TOOL**

(30) Priority: 22.01.2010 US 297423 P; 20.07.2010 JP 2010162989
(71) Applicant: OLYMPUS CORPORATION, Shibuya-ku Tokyo 151-0072 (JP)
(72) Inventor: KOBAYASHI, Masayuki, Tokyo 151-0072 (JP); OKAZAKI, Yoshiro, Tokyo 151-0072 (JP)
(74) Representative: Schicker, Silvia
(86) International application number: PCT/JP2011/050176
(87) International publication number: WO 2011/089935

(57) **Abstract**

A procedure of positioning a treatment part at various sites on an organ is facilitated, and the positioning thereof can be stably maintained for treatment with accurate positioning. A treatment instrument (1) includes a rigid rod-shaped insertion section (2) having a treatment part at a distal end thereof and a manipulating section (3) secured to a proximal end of the insertion section (2) and gripped and manipulated by an operator. The insertion section (2) includes a main body (8) extending in a straight line, a first curved portion (9) disposed at a distal end of the main body (8) and curved in one direction with respect to the main body (8), and a second curved portion (10) disposed on a distal side of the first curved portion (9) and curved in a direction opposite to the direction in which the first curved portion (9) is curved. The treatment part is inclined with respect to the longitudinal axis of the main body (8).

## Description

### {Technical Field}

The present invention relates to treatment instruments .

### {Background Art}

Conventionally known treatment instruments for treatment such as injection of, for example, a drug solution into an organ, such as the heart, or cauterization of the surface of tissue include flexible catheters for introduction into a patient's body through a forceps channel of an endoscope (see, for example, PTL 1). One known catheter is formed in the shape of a hollow tube and has an injection needle disposed at the distal end thereof and a syringe connected to the proximal end thereof and containing an injectant. Another known catheter has electrodes disposed at the distal end thereof and a cable routed therethrough for supplying power across the electrodes.

For example, for treatment such as injection of an injectant into the surface of an organ, namely, the heart, or cauterization thereof, an endoscope is introduced from under the xiphoid process through the skin into the pericardial cavity, a catheter is introduced from a distal end opening of the endoscope through the forceps channel, and the distal end of the catheter is extended from the forceps channel to apply an injection needle or electrodes-disposed at the distal end of the catheter to the surface of the heart.

### {Citation List}

### {Patent Literature}

{PTL 1} Publication of Japanese Patent No. 2793884 {Summary of Invention}

### {Technical Problem}

For injection into or cauterization of the surface of the heart, the injection needle or electrodes must be positioned by manipulating the proximal end of the endoscope, which is exposed outside the body. If the endoscope is a flexible endoscope, the flexibility of the insertion section makes it difficult to move the distal end of the insertion section so as to follow the manipulation of the proximal end, resulting in a problem in that it is difficult to treat with accurate positioning.
On the other hand, if the endoscope is a rigid endoscope, the distal end of the insertion section can be moved so as to follow the manipulation of the proximal end.

However, there is inconvenience that it is difficult for each part of the three-dimensional internal organs which have a curved surface of a convex outside to arrange the tip of an insertion part stably. That is, for a rigid endoscope, however, the distal end of the insertion section has only one point in contact with the surface of the organ because the insertion section does not deform. The positioning of the distal end at various sites must therefore be independently performed without a guide for positioning and the procedure of positioning is difficult. Especially in the case of the pulsating organs like the heart, if the tip of an insertion part is separated from the organs after the disposal of the one part, positioning to the following part is very difficult.

The present invention has been made in view of the above-described circumstances, and an object thereof is to provide a treatment instrument that facilitates a procedure of positioning a treatment part at various sites on an organ and that allows the positioning thereof to be stably maintained for treatment with accurate positioning.

### {Solution to Problem}

To achieve the above-described object, the present invention provides the following solutions.
The present invention provides a treatment instrument including a rigid rod-shaped insertion section having a treatment part at a distal end thereof and a manipulating section secured to a proximal end of the insertion section and gripped and manipulated by an operator. The insertion section includes a main body extending in a straight line, a first curved portion disposed at a distal end of the main body and curved in one direction with respect to the main body, and a second curved portion disposed on a distal side of the first curved portion and curved in a direction opposite to the direction in which the first curved portion is curved. The treatment part is inclined with respect to the longitudinal axis of the main body.

According to the present invention, the operator grips the manipulating section and inserts the insertion section into the patient's body. Because the insertion section is formed in the shape of a rigid rod, the operator can insert the insertion section while estimating the position of the treatment part at the distal end of the insertion section using the longitudinal axis of the main body as a measure of the insertion direction.

The first curved portion at the distal end of the insertion section is curved from the main body in one direction. That is, a convex portion is formed at the distal end of the insertion section and can be made to abut, for example, against an organ in the body. By making the convex portion abut against the organ, the main body is supported at two points, namely, the manipulating section at the proximal end of the insertion section and the convex portion. This stabilizes the position of the treatment part.

Because the treatment part is disposed at the distal end of the second curved portion curved in the direction opposite to the direction in which the first curved portion is curved, the treatment part is directed toward the surface of, for example, an organ located in front of the first curved portion. Because the treatment part is inclined with respect to the longitudinal axis of the main body, the treatment part is also inclined with respect to the surface of the organ. In particular, if the treatment part is an injection needle, it can be inserted in a direction at an angle that is not perpendicular or excessively shallow with respect to the surface of the organ, thus improving the ease of manipulation.

In addition, with the convex portion abutting the organ, the operator can rotate the manipulating section about the longitudinal axis of the main body to move the treatment part about the longitudinal axis of the main body such that the convex portion serves as a fulcrum. That is, the treatment part can be stably moved toward the organ to be treated, thus allowing treatment with accurate positioning at a plurality of sites.

In the present invention, the manipulating section may include a handle extending substantially along an extension of the longitudinal axis of the main body.
In this case, the longitudinal axis of the main body is oriented in the direction in which the forefinger to the little finger are arranged when the operator grips the handle. The operator can therefore easily exert insertion force on the insertion section merely by gripping the handle and applying force in the direction in which the fingers are arranged. In addition, the operator can rotate the insertion section about the longitudinal axis of the main body merely by bending the wrist of the hand gripping the handle.

In the above configuration, the manipulating section may include a switch, and the treatment part may be operated by manipulating the switch.
In this case, the operator can operate the treatment part by manipulating the switch while gripping the handle.

In the above configuration including the switch, the switch may be a lever disposed on the handle so as to be pivotable.
In this case, the operator can easily manipulate the treatment part by pivoting the lever with respect to the handle with the hand gripping the handle.

In the above configuration including the lever as the switch, the insertion section may be curved in a plane, and the lever may be disposed so as to be pivotable in another plane crossing the plane.
This allows the orientation of the back of the hand manipulating the lever to be different from the plane in which the treatment part is operated. For example, if the operator stands to the right of a patient lying spine and grips the handle in the right hand to insert the insertion section from under the xiphoid process into the pericardial cavity for therapy of the heart, the operator can easily conduct the procedure of inserting the insertion section with the back of the right hand facing upward.

In this state, the treatment part can be located at an appropriate angle with respect to the surface of the anterior wall of the left ventricle and can be easily operated by manipulating the lever. With respect to the state of the right hand at that time, the operator can easily bend the right wrist over a wide range. This ensures a wide range of movement of the treatment part, thus allowing treatment in a natural posture.

In the above configuration in which the insertion section is curved in a plane and the lever is disposed so as to be pivotable in another plane crossing the plane, the plane in which the insertion section is disposed may be perpendicular to the plane in which the lever is pivotable.
In this case, for the above example, the insertion section can be disposed in the vertical plane with the back of the right hand facing upward.

In the above configuration including the lever as the switch, the insertion section may be formed in the shape of a hollow tube and may have a flexible tube inserted therein so as to be movable in the longitudinal direction and having an injection needle attached to a distal end thereof, the treatment part may have an opening through which the injection needle is extended from or retracted into the distal end of the insertion section, and the lever may have a securing section for securing the tube and disposed so as to be movable in the longitudinal direction of the insertion section.

In this case, treatment can be easily conducted by moving the tube secured to the securing section through the manipulation of the lever to move the injection needle inserted in the insertion section in the longitudinal direction of the insertion section so that the injection needle is extended from the opening at the distal end of the insertion section and is inserted into the surface of, for example, an organ.

In the above configuration including the lever as the switch, the treatment part may have an opening that opens at the distal end of the insertion section, the insertion section may be formed in the shape of a hollow tube to accommodate a flexible catheter having a pair of electrodes attached to a distal end thereof such that the electrodes at the distal end are extended from the opening, and the switch may turn on and off a voltage supplied across the electrodes.

In this case, treatment such as cauterization or electric stimulation of the surface of, for example, an organ can be easily conducted by extending the pair of electrodes at the distal end of the catheter from the opening at the distal end of the insertion section, bringing the pair of extended electrodes into contact with the surface of the organ, and supplying a voltage across the electrodes through the manipulation of the switch.

In the present invention, the first curved portion of the main body may include a movable portion for changing the curvature of the first curved portion, and the manipulating section may include a manipulating member for operating the movable portion.
In this case, the curvature of the first curved portion can be changed at the movable portion by manipulating the manipulating member provided in the manipulating section to change the direction of the distal tip of the insertion section to a desired direction so that the treatment part approaches the surface of, for example, an organ at an appropriate angle.

In the present invention, the second curved portion of the main body may include a movable portion for changing the curvature of the second curved portion, and the manipulating section may include a manipulating member for operating the movable portion.
In this case, the curvature of the second curved portion can be changed at the movable portion by manipulating the manipulating member provided in the manipulating section to change the direction of the distal tip of the insertion section to a desired direction so that the treatment part approaches the surface of, for example, an organ at an appropriate angle.

### {Advantageous Effects of Invention}

With the present invention, it is possible to facilitate a procedure of positioning a treatment part at various sites on an organ and allow the positioning thereof to be stably maintained for treatment with accurate positioning.

### {Brief Description of Drawings}

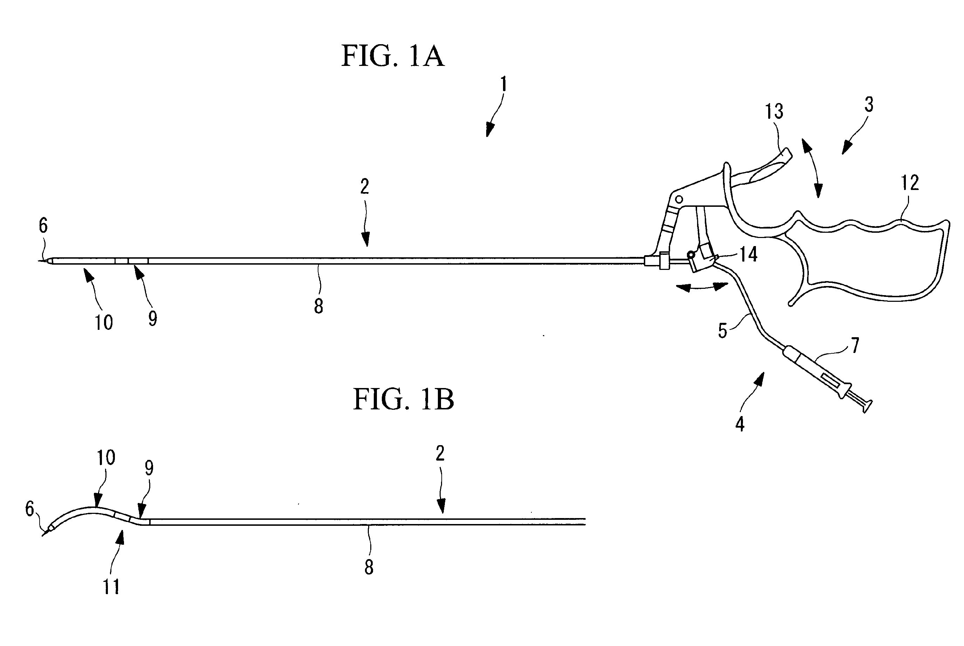

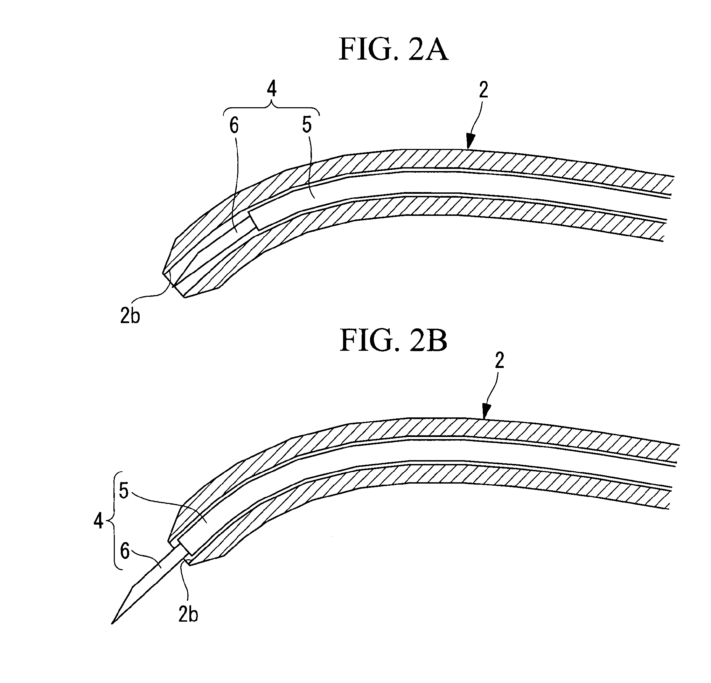

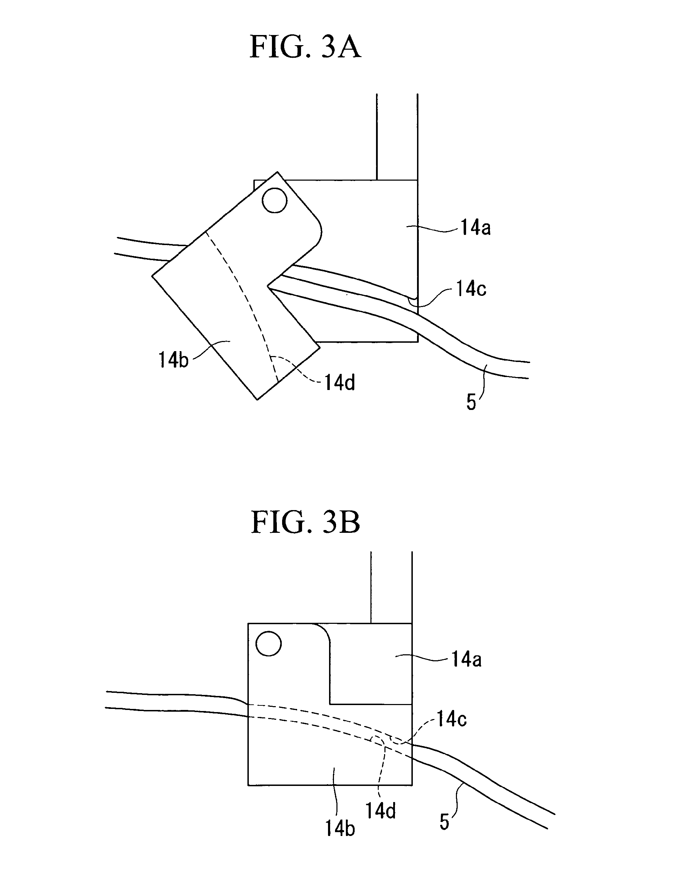

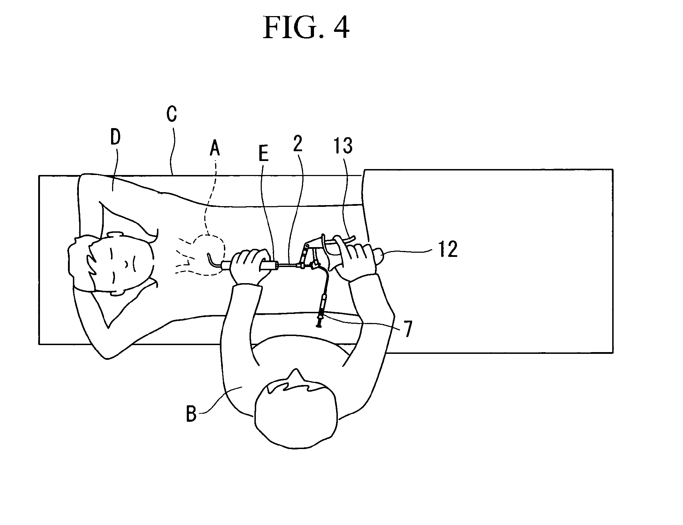

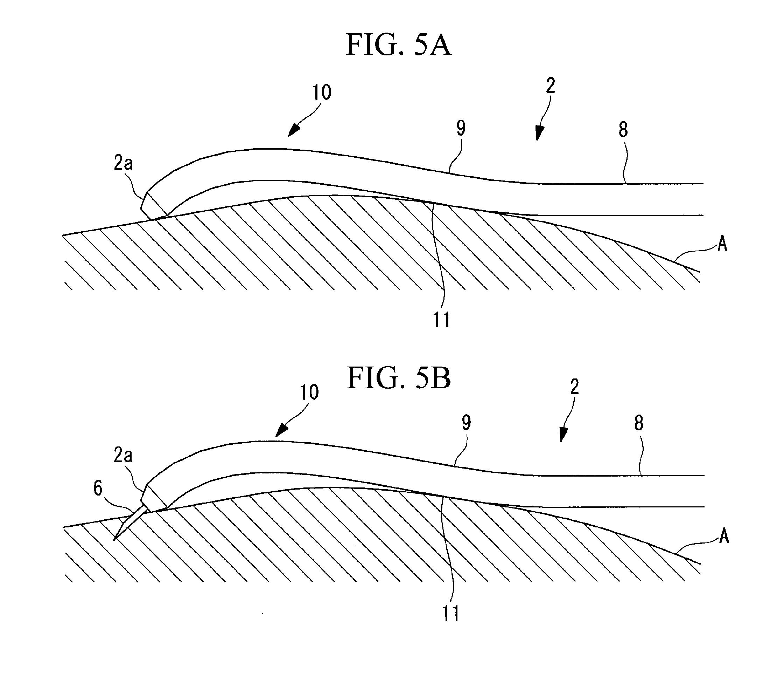

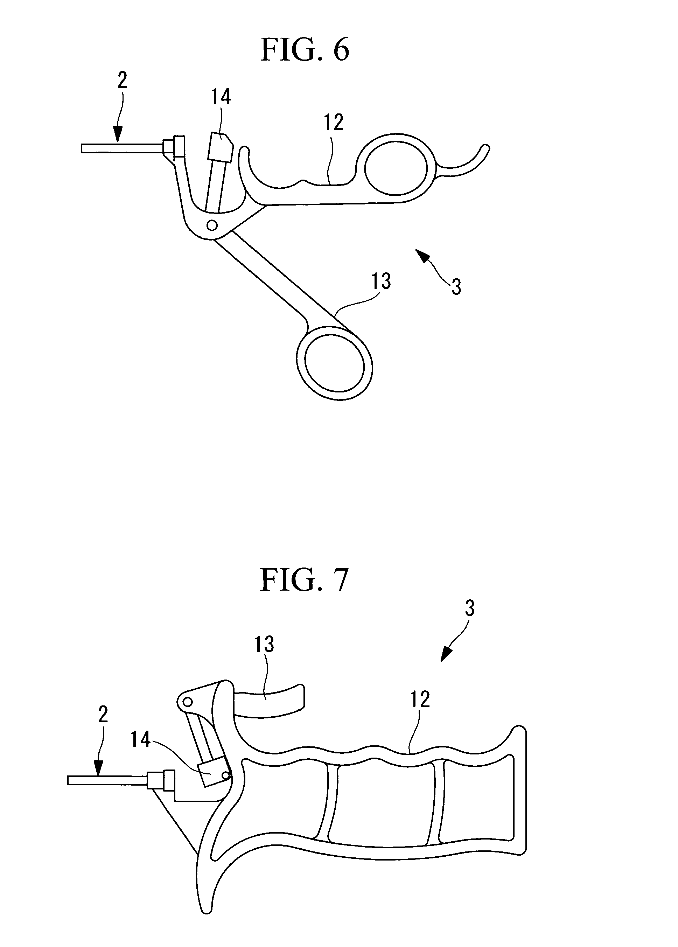

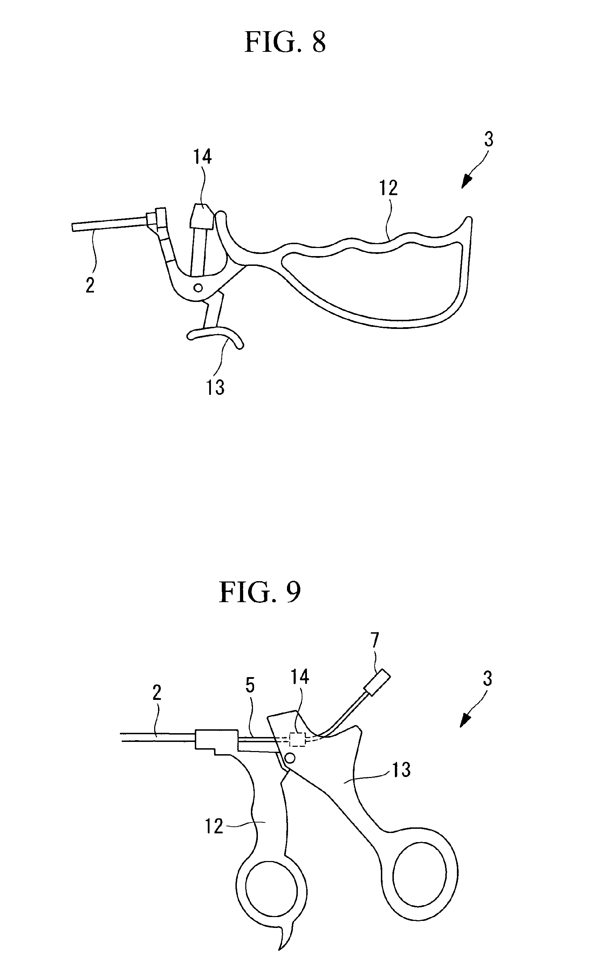

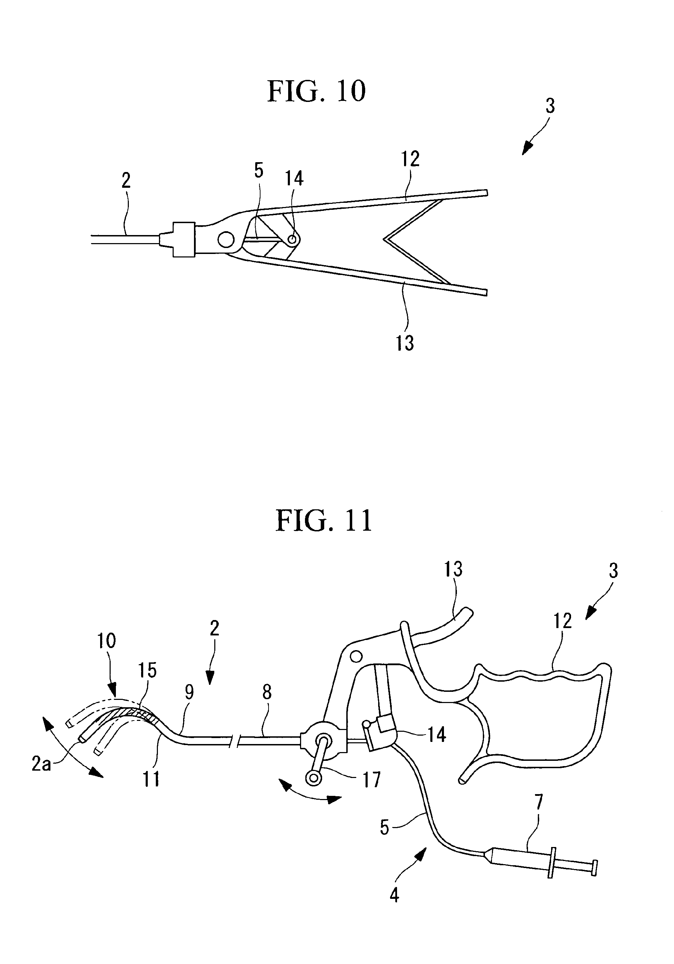

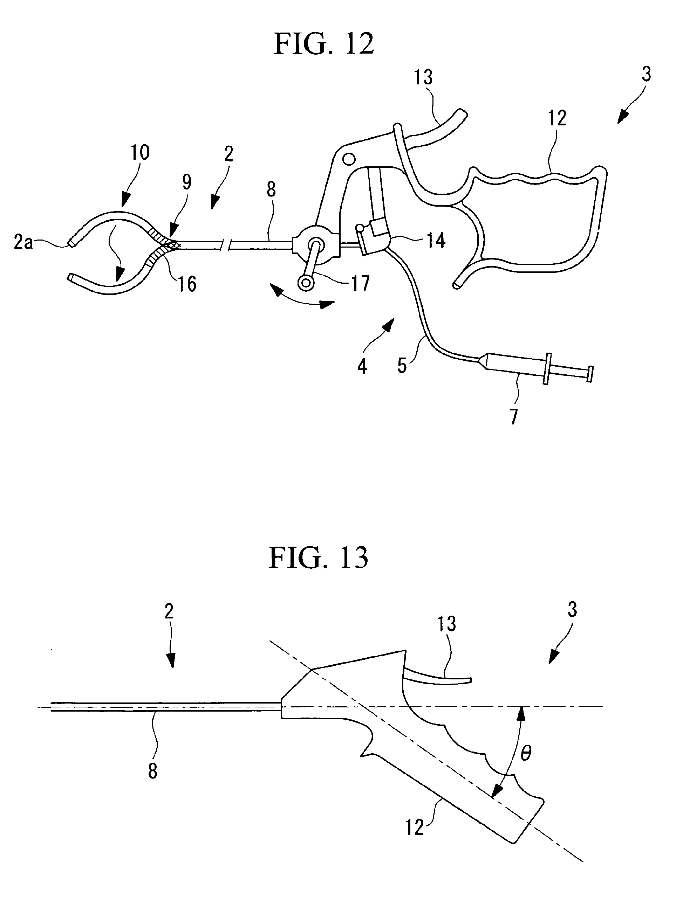

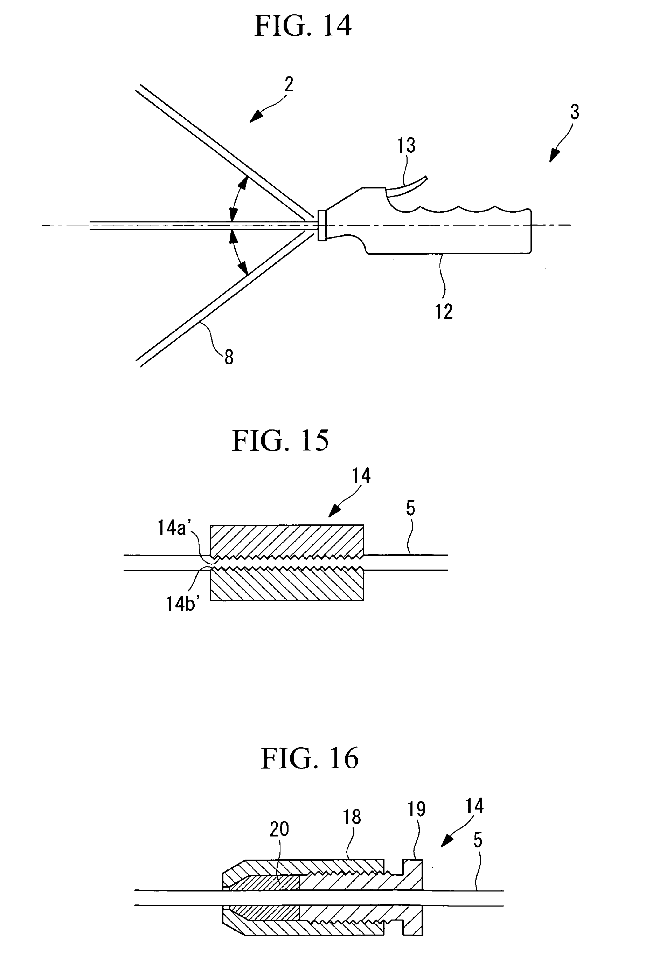

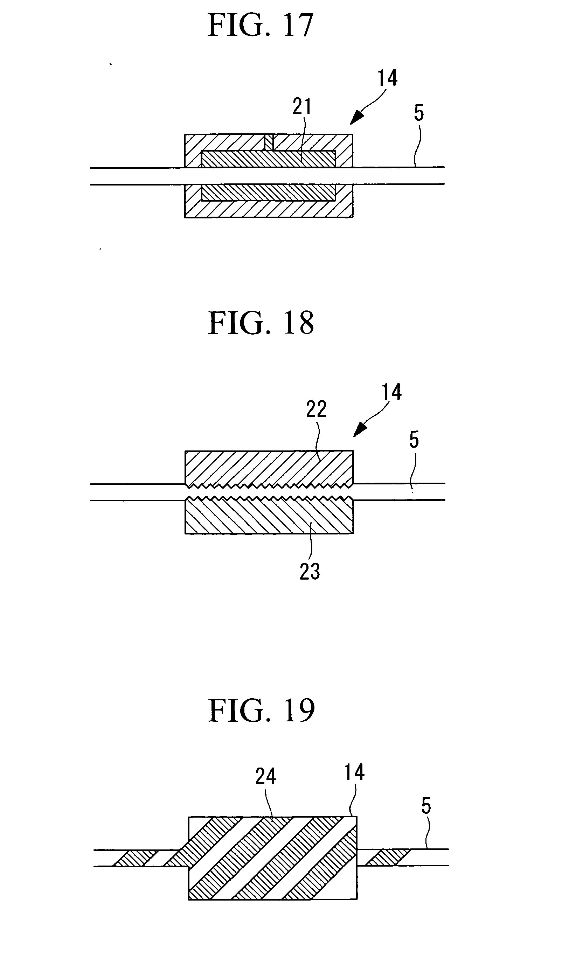

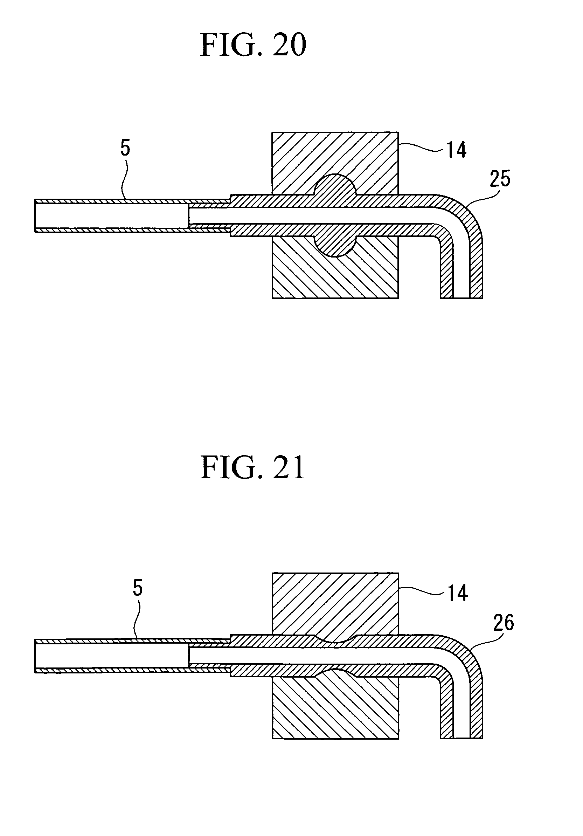

{Fig. 1A} Fig. 1A is a front view showing a treatment instrument according to an embodiment of the present invention.
{Fig. 1B} Fig. 1B is a side view of an insertion section of the treatment instrument in Fig. 1A.
{Fig. 2A} Fig. 2A is a partial longitudinal sectional view showing the internal structure of a distal end of the insertion section of the treatment instrument in Fig. 1A, where Fig. 2A shows the state where a treatment device is retracted.
{Fig. 2A} Fig. 2B is a partial longitudinal sectional view showing the internal structure of a distal end of the insertion section of the treatment instrument in Fig. 1A, where Fig. 2B shows the state where the treatment device is extended.
{Fig. 3A} Figs. 3A is a diagram showing a securing section provided on the treatment instrument in Fig. 1A, where Fig. 3A shows the state where the treatment device is not secured.
{Fig. 3B} Figs. 3B is a diagram showing a securing section provided on the treatment instrument in Fig. 1A, where Fig. 3B shows the state where the treatment device is secured.
{Fig. 4} Fig. 4 is a diagram illustrating heart therapy using the treatment instrument in Fig. 1A.
{Fig. 5A} Fig. 5A is a diagram showing the state where the distal end of the insertion section of the treatment instrument in Fig. 1A is brought into contact with the surface of an organ.
{Fig. 5B} Fig. 5B is a diagram showing the state where an injection needle is extended from the distal end of the insertion section of the treatment instrument in Fig. 1A and is inserted into the organ.
{Fig. 6} Fig. 6 is a diagram showing a modification of a handle of the treatment instrument in Fig. 1A.
{Fig. 7} Fig. 7 is a diagram showing a modification of the handle of the treatment instrument in Fig. 1A.
{Fig. 8} Fig. 8 is a diagram showing a modification of the handle of the treatment instrument in Fig. 1A.
{Fig. 9} Fig. 9 is a diagram showing a modification of the handle of the treatment instrument in Fig. 1A.
{Fig. 10} Fig. 10 is a diagram showing a modification of the handle of the treatment instrument in Fig. 1A.
{Fig. 11} Fig. 11 is a diagram showing a modification in which a movable portion is provided in a second curved portion of the treatment instrument in Fig. 1A.
{Fig. 12} Fig. 12 is a diagram showing a modification in which a movable portion is provided in a first curved portion of the treatment instrument in Fig. 1A.
{Fig. 13} Fig. 13 is a diagram showing a modification in which the angle between the insertion section and the handle of the treatment instrument in Fig. 1A is changed.
{Fig. 14} Fig. 14 is a diagram showing a modification in which the angle between the insertion section and the handle of the treatment instrument in Fig. 1A is variable.
{Fig. 15} Fig. 15 is a diagram showing a modification of the securing section of the treatment instrument in Fig. 1A.
{Fig. 16} Fig. 16 is a diagram showing a modification of the securing section of the treatment instrument in Fig. 1A.
{Fig. 17} Fig. 17 is a diagram showing a modification of the securing section of the treatment instrument in Fig. 1A.
{Fig. 18} Fig. 18 is a diagram showing a modification of the securing section of the treatment instrument in Fig. 1A.
{Fig. 19} Fig. 19 is a diagram showing a modification of the securing section of the treatment instrument in Fig. 1A.
{Fig. 20} Fig. 20 is a diagram showing a modification of the securing section of the treatment instrument in Fig. 1A.
{Fig. 21} Fig. 21 is a diagram showing a modification of the securing section of the treatment instrument in Fig. 1A.

### {Description of Embodiments}

A treatment instrument 1 according to an embodiment of the present invention and a treatment method using the treatment instrument 1 will be described below with reference to the drawings.
As shown in Fig. 1A, the treatment instrument 1 according to this embodiment includes a rigid rod-shaped insertion section 2 and a manipulating section 3 secured to the proximal end of the insertion section 2 and manipulated by the operator.

As shown in Figs. 2A and 2B, the insertion section 2 is formed as a hollow member so that a flexible treatment device 4 can be inserted into the insertion section 2 so as to be movable. For example, as shown in Figs. 2A and 2B, the treatment device 4 includes an elongated catheter 5, a rigid injection needle 6 attached to the distal end of the catheter 5, and a syringe 7 attached to the proximal end of the catheter 5 and containing, for example, a drug solution or stem cells.

As shown in Figs. 1A and 1B, the insertion section 2 includes a main body 8 formed in the shape of a straight rod extending from the proximal end toward the distal end, a first curved portion 9 disposed at the distal end of the main body 8 and curved in one direction, and a second curved portion 10 disposed on the distal side of the first curved portion 9 and curved in the direction opposite to the direction in which the first curved portion 9 is curved. The first curved portion 9 forms a shoulder portion 11 at the distal end of the main body 8.

The first curved portion 9 and the second curved portion 10 are formed by curving the main body 8, which extends from the proximal end in the shape of a straight rod, twice in the same plane (hereinafter referred to as "plane of curvature"). Accordingly, a distal tip (treatment part) 2a of the insertion section 2 on the distal side of the second curved portion 10 makes a predetermined angle with respect to the longitudinal axis of the main body 8. Here, the predetermined angle is, for example, 15° to 60°.

If a target organ A to be treated is the heart, and if the treatment is injection into the surface of the heart, the injection needle 6 cannot be inserted into the surface of the heart if the puncture angle of the injection needle 6 is insufficient and, on the other hand, may penetrate into the heart if the puncture angle is excessive. Therefore, the predetermined angle of the distal tip 2a with respect to the longitudinal axis of the main body 8 preferably falls within the above angular range.

The curvatures of the first curved portion 9 and the second curved portion 10 are set so that the injection needle 6, which is the rigid portion of the flexible treatment device 4 movable therethrough, can be smoothly moved. In particular, the radius of curvature of the second curved portion 10 is set to be lower than the radius of curvature of the outer surface of the target organ A to be treated. In this case, as shown in Fig. 5A, the second curved portion 10 can be separated from the surface of the target organ A with the first curved portion 9 remaining in contact with the surface of the target organ A, thus ensuring sufficient treatment space near the surface of the target organ A.

For example, if the target organ A is the heart (hereinafter referred to as "heart A"), sufficient treatment space can be ensured by bringing the first curved portion 9 of the insertion section 2 inserted between the heart A and the pericardium into contact with the surface of the heart A and pressing the pericardium with the second curved portion 10 in a direction away from the heart A. It is preferable, however, that the maximum separation distance between the main body 8 and the second curved portion 10 be limited to 30 mm because excessively expanding the space between the heart A and the pericardium could result in complications such as cardiac tamponade.

As shown in Fig. 1A, the manipulating section 3 includes a handle 12 gripped by the operator, such as a physician, and a lever (switch) 13 pivotably disposed on the handle 12. For example, the handle 12 is shaped and sized so as to be easily gripped in the right hand. The handle 12 is disposed so as to extend substantially along an extension of the longitudinal axis of the main body 8 of the insertion section 2. The handle 12 is disposed such that, when the operator grips the handle 12 in the right hand with an overhand grip, the main body 8 extends in the direction in which the forefinger to the little finger of the right hand are arranged.

The lever 13 is pivotably disposed at the position corresponding to the forefinger of the right hand gripping the handle 12. For insertion of the insertion section 2 in the longitudinal direction thereof, the operator can grip the handle 12 in all the five fingers and the palm with a powerful grip. For treatment with the treatment device 4, on the other hand, the operator can pivot the lever 13 with the forefinger while gripping the handle 12 in the four fingers other than the forefinger and the palm.

A securing section 14 is secured to the lever 13. The securing section 14 is movable in a direction toward or away from an opening (not shown) at the proximal end of the main body 8 as the lever 13 is pivoted.

As shown in Figs. 3A and 3B, the securing section 14 includes two members 14a and 14b that can be combined together. The securing section 14 allows the treatment device 4 to be secured by holding a certain position of the catheter 5 of the treatment device 4 between curved surfaces 14c and 14d of the members 14a and 14b, respectively. That is, as the lever 13 is pivoted with the certain position of the catheter 5 being secured to the securing section 14, the treatment device 4 is pushed or drawn in the longitudinal direction thereof so that the injection needle 6 at the distal end is extended from or retracted into a distal opening 2b of the insertion section 2.
In addition, as shown in Fig. 1A, the plane of pivoting of the lever 13 disposed on the manipulating section 3 extends in a direction perpendicular to the plane of curvature of the insertion section 2.

The operation of the thus-configured treatment instrument 1 according to this embodiment will be described below.
For treatment of the target organ A, for example, the heart, by using the treatment instrument 1 according to this embodiment, as shown in Fig. 4, an operator B stands to the right of a patient D lying spine on a bed C while holding the treatment instrument 1 by gripping the handle 12 in the right hand. The operator B then inserts the distal end of the insertion section 2 into the pericardial cavity through a sheath E passed from under the xiphoid process into the pericardial cavity substantially along the midline of the patient D in the cranial direction. That is, the operator B can easily guide the distal end of the insertion section 2 to the heart A by straightening out the wrist with the back of the right hand facing upward and pushing the handle 12, gripped in all the fingers and the palm with an overhand grip, in the direction toward the distal end of the insertion section 2. In some cases, the operator B grips the sheath E or the insertion section 2 in the left hand.

As shown in Fig. 5A, the distal tip 2a of the insertion section 2 guided into the pericardial cavity through the sheath E is brought into contact with the surface of the heart A. In this case, because the insertion section 2 has the shoulder portion 11 formed by the first curved portion 9 at the distal end of the straight-rod-shaped main body 8, the shoulder portion 11 is brought into contact with the surface of the heart A so that the insertion section 2 is stably supported at two points, namely, the contact portion and the handle 12 outside the body. The distal tip 2a of the insertion section 2 can then be located at different positions on the surface of the heart A by manipulating the position of the handle 12 and the angle of the main body 8 about the longitudinal direction thereof such that the point of contact between the heart A and the shoulder portion 11 serves as a fulcrum.

Subsequently, as shown in Fig. 5A, the distal tip 2a of the insertion section 2 is brought into contact with the surface of the heart A so that the insertion section 2 comes into contact with the surface of the heart A at two points, namely, the shoulder portion 11 formed by the first curved portion 9 and the distal tip 2a. This further stabilizes the orientation of the insertion section 2 for more precise treatment.
In this state, the operator B pushes the catheter 5 of the treatment device 4 by pulling the lever 13 disposed on the handle 12 with the forefinger of the right hand to move the securing section 14 in the direction toward the proximal end of the insertion section 2, thus extending the injection needle 6 at the distal end of the catheter 5 from the distal opening 2b of the insertion section 2, as shown in Fig. 5B.

Thus, the injection needle 6 is extended and punctured the surface of the heart A at an appropriate angle with respect to the surface of the heart A. The operator B can then inject, for example, a drug solution into the surface of the heart A by manipulating the syringe 7 with the left hand for proper treatment. In addition, the operator B can stably inject the drug solution into a plurality of intended sites on the surface of the heart A by repeatedly manipulating the lever 13 and the syringe 7 while changing the position and angle of the handle 12.

The insertion section 2 transmits heartbeats from the heart A to the hands of the operator B via the distal tip 2a of the insertion section 2 in contact with the surface of the heart A. In addition, the insertion section 2 is affected by the respiratory movement of the thorax and the diaphragm. Under such conditions where the operator B has difficulty in handling the insertion section 2 as he or she wishes, he or she must accurately perform treatment such as, for example, injection of drug solution or stem cells into a boundary region between a myocardial infarction site and a normal site in the left ventricle at intervals of several millimeters.

The treatment instrument 1 according to this embodiment provides the advantage of facilitating the procedure of positioning the distal tip 2a of the insertion section 2 at various positions on the target organ A and allowing the positioning thereof to be stably maintained for treatment with accurate positioning.

Although the treatment device 4 illustrated in this embodiment has the injection needle 6 at the distal end of the catheter 5, the treatment device used is not limited thereto; it may instead be an ablation catheter, for cauterizing the target organ A, having electrodes (not shown) at the distal end thereof.

In addition, a pair of electrodes (not shown) insulated from each other may be disposed at the distal tip 2a of the insertion section 2 to cauterize the injection hole of the injection needle 6 by means of radio-frequency current after the injection of, for example, drug solution or stem cells through the catheter 5, thereby preventing leakage of the drug solution or stem cells. As the means for cauterizing the injection hole, it may be cauterized by light irradiation instead of using electrodes. In addition, a cooling mechanism (not shown) for refluxing a coolant, such as physiological saline, through the insertion section 2 may be provided to prevent an excessive rise in the temperature of the target site during cauterization.

In addition, although the insertion section 2 illustrated has a hollow structure into which the treatment device 4 is inserted so as to be movable, a solid insertion section may be used instead. That is, a rigid treatment device having a treatment part, such as electrodes (not shown), at the distal end thereof and having a curved shape that comes into contact with the target organ A at two points may itself be used as the insertion section 2. In this case, the securing section 14 is unnecessary, and the lever 13 may be replaced with, for example, a pushbutton switch.

In addition, although the distal end of the insertion section 2 is preferably located on an extension of the main body 8, the position is not limited thereto; it may be freely set depending on, for example, the type and shape of the target organ A and the angle at which the treatment device 4 is extended.

In addition, the shape of the manipulating section 3 is not limited to the structure shown in Fig. 1A; a manipulating section 3 having any shape can be used, as illustrated in Figs. 6 to 10. The manipulating section 3 in Fig. 6 is the "scissors type," which allows delicate manipulation as does conventional forceps, and is configured so that the thumb is secured to the lever 13 for manipulating the treatment device 4 with the handle 12 gripped with an overhand grip. The manipulating section 3 in Fig. 7 includes a handle 12 to grip with an overhand grip similar to the manipulating section 3 in Fig. 1A, but the securing section 14 is located at a different position. The manipulating section 3 in Fig. 8 includes a handle 12 to grip with an overhand grip similar to the manipulating section 3 in Fig. 1A, but the lever 13 is manipulated with the thumb.

The manipulating section 3 in Fig. 9 includes a "scissors type" handle 12 similar to the manipulating section 3 in Fig. 6, but the handle 12 and the lever 13 are reversed in position so that the direction of the hand gripping the handle 12 differs from the overhand grip of the manipulating section 3 in Fig. 6.
The manipulating section 3 in Fig. 10 includes a lever 13 built into the handle 12. The operator B can manipulate the treatment device 4 by gripping the handle 12 and the lever 13 in the entire palm for improved grip stability.

In addition, although the entire insertion section 2 is composed of a rigid member in this embodiment, as shown in Figs. 11 and 12, a movable portion 15 or 16 may instead be provided near the distal end of the insertion section 2. In the example shown in Fig. 11, the movable portion 15 is provided in the second curved portion 10 so as to be variable in curvature, and a manipulating member 17 for operating the movable portion 15 is provided on the manipulating section 3.

In the example shown in Fig. 12, the movable portion 16 is provided in the first curved portion 9 so as to be variable in curvature, and a manipulating member 17 for operating the movable portion 16 is provided on the manipulating section 3. This allows the movable portion 15 or 16 to be operated to change the angle of the distal tip 2a of the insertion section 2 and therefore the angle at which the treatment device 4 is extended for more complicated treatment of the target organ A.

In addition, it may be difficult to manipulate the treatment instrument 1 while keeping the hands straightened out, for example, in the case of a prolonged surgical procedure. Accordingly, as shown in Fig. 13, the angle θ between the longitudinal axis of the main body 8 of the insertion section 2 and the handle 12 may be set to a certain angle appropriate for manipulation so that the operator B does not easily get tired, for improved ease of manipulation. Alternatively, as shown in Fig. 14, the angle between the longitudinal axis of the main body 8 and the handle 12 may be freely variable with, for example, a ball-link mechanism (not shown).

In addition, the plane of pivoting of the lever 13 is perpendicular to the plane of curvature of the insertion section 2 in the example shown in Fig. 1A. This angle is appropriate for approach to the anterior wall of the left ventricle; instead, for approach to the posterior wall of the left ventricle or another site, any other angle appropriate for the target site to be approached may be set. In addition, the angle between the insertion section 2 and the handle 12 may be freely adjustable so that an appropriate angle can be set depending on, for example, the target organ A.

The treatment device 4 used can be, for example, a disposable puncture needle, an ablation catheter, a tube, a balloon, a laser therapy device, an optical fiber, or an ultrasonic probe.
Although the securing section 14, for securing and advancing/retracting the treatment device 4, illustrated in Figs. 3A and 3B has the curved surfaces 14c and 14d between which the treatment device 4 is held, it may instead be held between flat surfaces or curved surfaces on which, for example, rubber is laminated for increased friction.

In addition, the securing section 14 is not limited to the above structure; the structures shown in Figs. 15 to 21 may be used instead.
In the example shown in Fig. 15, the catheter 5 of the treatment device 4 is held between contact surfaces 14a' and 14b' having irregularities.
In the example shown in Fig. 16, a cylindrical sleeve 20 formed of silicone rubber is disposed between a housing 18 and a plug 19 through which the catheter 5 of the treatment device 4 is passed and is deformed by tightening the plug 19 into the housing 18 so that the inner diameter of the sleeve 20 becomes smaller, thus securing the catheter 5 of the treatment device 4.

In the example shown in Fig. 17, a box-shaped securing section 14 through which the catheter 5 is passed is filled with an adhesive 21 to bond and secure the catheter 5.
In the example shown in Fig. 18, the catheter 5 is held between magnets 22 and 23 and is secured by magnetic attraction.
In the example shown in Fig. 19, the catheter 5 is secured to the securing section 14 with an adhesive tape 24.

In addition, as shown in Figs. 20 and 21, an adaptor 25 or 26 having a protrusion or recess may be secured to the catheter 5, and the catheter 5 may be secured to the securing section 14 by fitting the protrusion or recess to a corresponding recess or protrusion of the securing section 14.
In addition, although the case where the operator B grips the handle 12 in the right hand has been illustrated, the handle 12 may instead be configured to be suitable for the operator B to grip in the left hand while standing to the left of the patient D lying spine on the bed C.

### {Reference Signs List}

- B: operator
- 1: treatment instrument
- 2: insertion section
- 2a: distal tip (treatment part)
- 2b: opening
- 3: manipulating section
- 5: catheter (tube)
- 6: injection needle
- 8: main body
- 9: first curved portion
- 10: second curved portion
- 12: handle
- 13: lever (switch)
- 14: securing section
- 15, 16: movable portion
- 17: manipulating member

## Claims

1. A treatment instrument comprising:
a rigid rod-shaped insertion section having a treatment part at a distal end thereof; and
a manipulating section secured to a proximal end of the insertion section and gripped and manipulated by an operator;
wherein the insertion section includes a main body extending in a straight line, a first curved portion disposed at a distal end of the main body and curved in one direction with respect to the main body, and a second curved portion disposed on a distal side of the first curved portion and curved in a direction opposite to the direction in which the first curved portion is curved; and
the treatment part is inclined with respect to the longitudinal axis of the main body.

2. The treatment instrument according to Claim 1, wherein the manipulating section includes a handle extending substantially along an extension of the longitudinal axis of the main body.

3. The treatment instrument according to Claim 2, wherein
the manipulating section includes a switch; and
the treatment part is operated by manipulating the switch.

4. The treatment instrument according to Claim 3, wherein the switch is a lever disposed on the handle so as to be pivotable.

5. The treatment instrument according to Claim 4, wherein
the insertion section is curved in a plane; and
the lever is disposed so as to be pivotable in another plane crossing the plane.

6. The treatment instrument according to Claim 5, wherein the plane in which the insertion section is disposed is perpendicular to the plane in which the lever is pivotable.

7. The treatment instrument according to Claim 4, wherein
the insertion section is formed in the shape of a hollow tube and has a flexible tube inserted therein so as to be movable in the longitudinal direction, the tube having an injection needle attached to a distal end thereof;
the treatment part has an opening through which the injection needle is extended from or retracted into the distal end of the insertion section; and
the lever has a securing section for securing the tube and disposed so as to be movable in the longitudinal direction of the insertion section.

8. The treatment instrument according to Claim 4, wherein
the treatment part has an opening that opens at the distal end of the insertion section;
the insertion section is formed in the shape of a hollow tube and accommodates a flexible catheter having a pair of electrodes attached to a distal end thereof such that the electrodes at the distal end are extended from the opening; and
the switch turns on and off a voltage supplied across the electrodes.

9. The treatment instrument according to Claim 1, wherein
the first curved portion of the main body includes a movable portion for changing the curvature of the first curved portion; and
the manipulating section includes a manipulating member for operating the movable portion.

10. The treatment instrument according to Claim 1, wherein
the second curved portion of the main body includes a movable portion for changing the curvature of the second curved portion; and
the manipulating section includes a manipulating member for operating the movable portion.
